(19) **Europäisches Patentamt / European Patent Office / Office européen des brevets**

(11) **EP 1 400 240 B1**

(12) **EUROPEAN PATENT SPECIFICATION**

(45) Date of publication and mention
of the grant of the patent:
**21.04.2010 Bulletin 2010/16**

(51) Int Cl.:
*A61K 9/70* (2006.01)      *A61K 31/196* (2006.01)
*A61K 45/00* (2006.01)     *A61K 47/10* (2006.01)
*A61K 47/12* (2006.01)     *A61K 47/18* (2006.01)
*A61K 47/32* (2006.01)     *A61P 29/00* (2006.01)

(21) Application number: **02724672.7**

(22) Date of filing: **02.05.2002**

(86) International application number:
**PCT/JP2002/004382**

(87) International publication number:
**WO 2002/098396 (12.12.2002 Gazette 2002/50)**

(54) **PERCUTANEOUSLY ABSORBABLE PATCHES**

PERKUTAN RESORBIERBARE PFLASTER

TIMBRES A ABSORPTION PAR VOIE PERCUTANEE

(84) Designated Contracting States:
**AT BE CH CY DE DK ES FI FR GB GR IE IT LI LU MC NL PT SE TR**

(30) Priority: **31.05.2001 JP 2001164065**

(43) Date of publication of application:
**24.03.2004 Bulletin 2004/13**

(73) Proprietor: **Hisamitsu Pharmaceutical Co., Inc.**
**Tosu-shi, Saga 841-0017 (JP)**

(72) Inventors:
• **TAKADA, Y.,**
**c/o HISAMITSU PHARMACEUTICAL CO.,INC.**
**Saga 841-0017 (JP)**
• **TANAKA, K.,**
**c/o HISAMITSU PHARMACEUTICAL CO.,INC.**
**Saga 841-0017 (JP)**
• **KURITA, T.,**
**c/o HISAMITSU PHARMACEUTICAL CO.,INC.**
**Saga 841-0017 (JP)**

• **IKEURA, Y.,**
**c/o HISAMITSU PHARMACEUTICAL CO.,INC.**
**Saga 841-0017 (JP)**

(74) Representative: **Cresswell, Thomas Anthony**
**J.A. Kemp & Co.**
**14 South Square**
**Gray's Inn**
**London**
**WC1R 5JJ (GB)**

(56) References cited:
EP-A- 0 285 181      EP-A- 0 607 434
EP-A- 0 634 179      EP-A1- 0 788 794
EP-A1- 0 879 597     EP-A2- 0 189 861
WO-A1-01/05381       GB-A- 2 273 044
JP-A- 4 217 925      JP-A- 5 155 762
JP-A- 9 208 463      JP-A- 9 208 542
JP-A- 61 012 621

• SANTUS, BAKER: "Transdermal enhancer in patent literature" JOURNAL OF CONTROLLED RELEASE, vol. 25, 27 May 1993 (1993-05-27), XP000361364

**Description**

Technical Field

This invention relates to a percutaneous absorbable patch which enhances percutaneous absorption of anti-inflammatory agent in the form of a salt and is able to contribute in more effective treatment.

Background Art

Treatment by a percutaneously applicable preparation of anti-inflammatory agent has been carried out from ancient times and many preparations such as cream, ointment, liquid, poultice, plaster, etc. have been placed on the market already. In addition, for the purpose of developing a preparation which is expected to have more effective curative effect, studies have been carried out broadly. However, it is not easy to deliver an effective amount of drug to the site of the inflammation via skin which has a barrier function for preventing the invasion of foreign body and the drug is limited to that having a property of being easily absorbed with the skin such as a free substance. Among the anti-inflammatory agents, there are many substances which are in the form of a salt and, since the skin achieves stronger barrier function to anti-inflammatory agents in the form of a salt as such, permeability of the drug is low and a sufficient pharmaceutical effect cannot be expected. Therefore, various investigations have been carried out to enhance the percutaneous absorbability. For example, in JP-B-07-47535, there is a proposal in which an organic acid which is strongly acidic than an acidic drug is added to make the drug a free form whereby percutaneous absorption is enhanced. However, as a result of making the drug into a free form, there are problems such as lowering of stability of the drug, irritation of the skin by the organic acid added thereto, lowering of physical property of the base, etc. In JP-A-05-155762, there is a proposal for a patch containing NSAIDS (nonsteroidal anti-inflammatory drugs) having a high solubility in triethylene glycol and an absorption enhancing composition such as triethylene glycol and terpene. However, absorption enhancing action of terpene for anti-inflammatory agent in the form of a salt is low and a sufficient pharmaceutical effect cannot be expected.

In JP-B-03-67043, there is proposed a patch where metal acetate or the like is contained so as to enhance the solubility of the drug having a metal salt of carboxyl group in a molecule but that is a proposal for enhancing the stability of the preparation and the percutaneous absorbability for the curative effect is not satisfactory.

On the other hand, there are investigations for enhancing the percutaneous absorbability by the use of an absorption enhancer. For example, in JP-A-09-48739, there is a proposal for a patch of an anti-inflammatory agent for external application where a nonionic surface-active agent having an unsaturated aliphatic hydrocarbon group is used as an absorption enhancer. However, there is no absorption enhancing effect for an anti-inflammatory agent in the form of a salt.

In JP-A-10-512245, although there is proposed a percutaneous absorption enhancing composition for a drug which is unstable to acid using a functional derivative of fatty acid the drug which is unstable to acid mentioned there is a compound having an $\alpha,\beta$-unsaturated ketone.

In WO 01/05381, there is proposed a percutaneously absorbable preparation where an addition salt compound of a basic substance is compounded whereby percutaneous absorbability of an acidic drug in the form of a salt is enhanced. However, even in such a percutaneously absorbable preparation, there are problems that percutaneous absorbability of the acidic drug in the form of a salt is not sufficient and that crystallization of the drug happens upon long period preservation whereby the percutaneous absorbability lowers.

Disclosure of the Invention

The present invention has been carried out to solve the above-mentioned problems in the prior art, and its object is to provide a percutaneously absorbable patch where the percutaneous absorbability of an anti-inflammatory agent in the form of a salt is enhanced and, further, stability thereof is enhanced.

Brief Description of the Invention

Fig. 1 shows the result of a skin permeation test using hairless mice for the percutaneously absorbable patches of Example 9, Example 25 and Comparative Examples 1 to 3.
Fig. 2 shows the result of a skin permeation test using hairless mice for the percutaneously absorbable patches of Example 3, Example 4, Comparative Example 4 and Comparative Example 5.

Best Mode for Carrying Out the Invention

In the present invention, as a result of intensive studies for solving the above-mentioned problems, it has been found that, when a salt of ammonium compound, an absorption enhancer as defined herein and/or a solubilizing agent as defined herein are/is compounded with an adhesive substrate layer containing an anti-inflammatory in the form of a salt, percutaneous absorbability of the anti-inflammatory agent is significantly improved and there is obtained a percutaneously absorbable patch in which, even if preserved for a long period, crystals of the drug are not separated out but stability is improved whereupon the present invention has been achieved.

Thus, the present invention provides a percutaneously absorbable patch having an adhesive substrate layer which contains an anti-inflammatory agent in the form of a salt, a salt of an ammonium compound, a absorption enhacer chosen from an unsaturated fatty acid, oleyl alcohol and polyoxyethylene ether and/or a solubilizing agent selected from the group consisting of a polyhydric alcohol, a nonionic surface-active agent and crotamiton.

The present invention will now be illustrated in detail as follows.

With regard to the anti-inflammatory agent in the form of a salt in accordance with the present invention, there is no particular limitation so far as it is acceptable as a drug. In a preferred embodiment, the anti-inflammatory agent is an acidic drug. Examples of a salt of the anti-inflammatory agent in the form of a salt are alkaline metal salt, alkaline earth metal salt, aluminum salt and tromethamine salt, and examples of the specific drug are sodium salicylate, sulpyrine, amfenac sodium, diclofenac sodium, diclofenac potassium, loxoprofen sodium, tolmetin sodium, lobenzarit disodium, ketorolac tromethamine, ketoprofen sodium, ibuprofen sodium, felbinac sodium, flurbiprofen sodium, indomethacin sodium, zomepirac sodium, aluminum flufenamate, fenoprofen calcium, bromfenac sodium, hydrocortisone sodium succinate, hydrocortisone sodium phosphate, dexamethasone sodium phosphate, dexamethasone sodium m-sulfobenzoate, betamethasone sodium phosphate, prednisolone sodium succinate, prednisolone sodium phosphate, methylprednisolone sodium succinate and prasterone sodium sulfate.

A preferred anti-inflammatory agent in the form of a salt is a salt of diclofenac, in particular diclofenac sodium or diclofenac potassium.

With regard to such anti-inflammatory agents in the form of a salt, one of them may used solely or two or more may be used jointly. There is no particular limitation for the amount of such an anti-inflammatory agent in the form of a salt with a percutaneous absorbable patch so far as it is an amount of achieving the pharmacological effect and it is usually within a range of 0.1 to 40 % by mass or, preferably, 0.5 to 30% by mass.

The salt of the ammonium compound in the present invention is a compound where a salt is formed by addition of another substance to an ammonium compound and, with regard to the ammonium compound, a Lewis base is preferred. A preferred one is that where a substance having an electron-deficient substance such as Lewis acid or a substance being able to form an electron-deficient system such as a halide is added to an electron-excessive part of a Lewis base whereby a salt comprising a cationic part and an anionic part is formed. The anionic part of the formed addition salt maybe organic such as carboxylic acid or sulfonic acid or may be inorganic such as halogen ion or phosphate, carbonate or sulfate ion and there is no particular limitation therefor so far as it is pharmaceutically acceptable. With regard to the salt of ammonium compound of the present invention, although a water-soluble one is preferred, it is not limited to the water-soluble one only.

With regard to the preferred salt of ammonium compound of the present invention, it may be an inorganic one such as an ammonium halide or may be an organic one such as a primary, secondary, tertiary or quaternary ammonium salt. Examples of the preferred salt of ammonium compound are a water-soluble salt of ammonia, dimethylamine, diethylamine, trimethylamine, monoethanolamine, diethanolamine, triethanolamine, etc.; a water-soluble salt of a primary, secondary or tertiary alkylamine or alkanolamine; a water-soluble quaternary ammonium salt; a water-soluble salt having a pyridinium group; and a water-soluble salt having a pyrrolidinium salt. To be morespecific, ammonium chloride, dimethylamine hydrochloride, diethylamine hydrochloride, 2-ethylhexylamine hydrochloride, n-dodecyl trimethylammonium chloride, benzalkonium chloride, tetramethylammonium chloride, n-hexadecylpyridinium chloride, triethanolamine hydrochloride, benzethonium chloride, domiphen bromide, nonylamine hydrochloride, choline hydrochloride, choline phosphate, cetylpyridinium chloride, methylrosaniline chloride, arginine hydrochloride, lysine hydrochloride, carbachol, hydroxyquinoline sulfate, may be listed as preferred examples. Particularly preferred are ammonium chloride and/or diethylamine hydrochloride.

With regard to those salt of ammonium compounds, one of themmaybe used solely or two ormore thereof may be used jointly. Although there is no particular limitation for the amount of the salt of ammonium compound with the percutaneously absorbable patch, a range of 0.5-fold mol to 7-fold mol to the anti-inflammatory agent in the form of a salt is usually preferred. When compounding is carried out within such a range, a highly percutaneous absorbability of the anti-inflammatory agent is achieved. Incidentally, when the amount is less than 0.5-fold mol, percutaneous absorbability of the anti-inflammatory agent is not sufficient and a sufficient pharmaceutical effect is not achieved. When it is more than 7-fold mol, although a sufficient percutaneous absorbability of the anti-inflammatory agent is achieved, solubility of the ammonium compound of the salt in the substrate becomes poor and lowering of the properties of the preparation is resulted. That is not preferred.

The absorption enhancer used in the present invention is an unsaturated fatty acid or a derivative of unsaturated fatty acid is preferred.

Examples of the unsaturated fatty acid are lauroleic acid, oleic acid, petroselinic acid, gadoleic acid, erucic acid, linoleic acid and linolenic acid and, among them, oleic acid is particularly preferred.

With regard to the derivative of unsaturated fatty acid, derivative of oleic acid are preferred. Examples thereof are oleyl alcohol, ethyl oleate, polyoxyethylene oleyl ether and oleamide and, among them, oleic alcohol and ethyl oleate are particularly preferred.

Preferred unsaturated fatty acids or derivatives thereof are oleic acid and/or oleic alcohol.

With regard to those absorption enhancers, one of them may be used solely or two or more may be used jointly. With regard to the amount of those absorption enhancers with the percutaneously absorbable patch, although there is no particular limitation so far as it is an amount of showing an absorption enhancing action for the anti-inflammatory agent in the form of a salt, it is within a range of usually 0.5 to 15% by mass or, preferably, 0.7 to 10% by mass. When compounding is done within such a range, a highly percutaneous absorbability of the anti-inflammatory agent is achieved. When the amount is less than 0.5% by mass, the percutaneous absorbability of the anti-inflammatory agent is not sufficient and a sufficient pharmaceutical effect is not achieved. When it is more than 15% by mass, although a sufficient percutaneous absorbability of the anti-inflammatory agent is achieved, solubility of the absorption enhancer in the substrate becomes poor and lowering of the properties of the preparation takes place whereby the outcome is not favorable.

The solubilizing agent used in the present invention is selected from polyhydric alcohol, a nonionic surface-active agent and crotamiton. In one embodiment, a preferred solubilizing agent is crotamiton.

Examples of the polyhydric alcohol are ethylene glycol, propylene glycol, diethylene glycol, dipropylene glycol, triethylene glycol, 1,3-butanediol, hexylene glycol, pentanediol, polypropylene glycol, polyethylene glycol, glycerol, 1,2,6-hexanetriol and pentaerythritol and, among them, dipropylene glycol, triethylene glycol, polyethylene glycol are preferred.

Examples of the nonionic surface-active agent are polyoxyethylene oleyl ether, polyoxyethylene lauryl ether, polyethylene glycol monostearate, polyoxyethylene polyoxypropylene cetyl ether, polyoxyethylene polyoxypropylene decyl tetradecyl ether, polyoxyethylene cetyl ether, polyoxyethylene nonyl phenyl ether, polyoxyethylene hydrogenated castor oil and polyoxyethylene sorbitan monooleate and, among them, polyoxyethylene lauryl ether and polyoxyethylene sorbitan monooleate are preferred.

Each of those solubilizing agent may be used solely or two or more thereof may be used jointly. Although there is no particular limitation for the amount of such a solubilizing agent to the percutaneously absorbable patch so far as it is an amount for showing a dissolving action to the anti-inflammatory agent, it is within a range of usually 0.5 to 20% by mass or, preferably, 0.7 to 15% by mass. When compounding is carried out within such a range, the anti-inflammatory agent in the form of a salt is able to be compounded with the percutaneously absorbable patch in a high concentration and a highly percutaneous absorbability can be maintained for long time. When the amount is less than 0.5% by mass, solubility of the anti-inflammatory agent in the form of a salt in the percutaneously absorbable patch becomes low and, upon preservation for long time, crystals of the drug are separated out and the percutaneous absorbability lowers whereby the outcome is not preferred. When it is more than 20% by mass, although solubility of the anti-inflammatory agent in the form of a salt becomes high, solubility of the anti-inflammatory agent in the form of a salt in the substrate becomes bad, property of the preparation lowers and the percutaneous absorbability lowers whereby the outcome is not favorable.

The optimum ratio of the anti-inflammatory agent in the form of a salt, the salt of an ammonium compound, the absorption enhancer and the solubilizing agent in the percutaneously absorbable patch of the present invention in terms of the ratio by mass to 1 part of the anti-inflammatory agent in the form of a salt is 0.08 to 5 part (s) of the ammonium compound, 0.03 to 10 part(s) of the absorption enhancer and 0.1 to 10 part (s) of the solubilizing agent. When the absorption enhancer and the solubilizing agent are used together, the optimum compounding ratio of the solubilizing agent in terms of ratio by mass to 1 part of the absorption enhancer is 0.05 to 15 part(s). When the ratio is made as such, it is now possible to prepare a percutaneously absorbable patch where adhesion to the skin is good, percutaneous absorbability of the anti-inflammatory agent is high and stability is good.

With regard to the adhesive substrate used in the percutaneously absorbable patch of the present invention, one or more polymer(s) selected from a styrene-isoprene-styrene block copolymer, polyisobutylene and an adhesive of an acrylate type are listed.

Specific examples of the styrene-isoprene-styrene block copolymer are Cariflex TR-1107, TR-1111, TR-1112 or TR-1117 (trade name; Shell Kagaku K. K.), Quintac 3530, 3570C or 3421 (trade name; Nippon Zeon Co., Ltd.), JSR SIS-5000 or 5002 (Japan Synthetic Rubber Co., Ltd.), Solprene 428 (trade name, Phillip Petroleum K. K.). Its compounding amount is 10 to 40% by mass or, preferably, 15 to 35% by mass of the percutaneously absorbable patch and, when the compounding amount is made as such, there are big improvements in adhesive property, adhesion to the skin for long time, percutaneous absorbability of the drug, pain upon peeling off, skin rash. Incidentally, when the compounding amount is less than 10% by mass, cohesive force and shape-holding property lower and the outcome is not favorable. When it is more than 40% by mass, cohesive force of the substrate increases resulting in reduction of adhesive force and heterogeneity of the patch and the outcome is not favorable.

Specific examples of polyisobutylene are Oppanol B-3, B-10, B-15, B-50, B-100 and B-200 (trade names; BASF), Vistanex LM-MS, LM-MH, MML-80, LLM-100, LLM-120andLLM-140 (tradenames; Exxon Chemical K. K.), Tetrax 3T, 4T, 5T and 6T (trade names; Nippon Petrochemical Co., Ltd.). Its compounding amount is 6 to 40% by mass or, preferably, 7 to 20% by mass of the percutaneously absorbable patch and, when the compounding amount is made as such, there are big improvements in tackiness, adhesion to the skin for long time, percutaneous absorbability of the drug, pain upon peeling off, skin rash. Incidentally, when the compounding amount is less than 6% by mass, tackiness and adhesion to the skin for long time lower and pain upon peeling off and skin rash increase whereby the outcome is not favorable. When it is more than 40% by mass, shape-holding property lowers and stickiness increase whereby the outcome is not favorable.

The adhesive of an acrylate type comprises a copolymer comprising at least two members of butyl acrylate, 2-ethylhexyl acrylate, vinyl acetate, methacrylate, hydroxyethyl acrylate, glycidyl methacrylate, methoxyethyl acrylate, acrylic acid, and specific examples thereof are DURO-TAK 87-2097, 87-2194, 87-2196, 87-2287, 87-2516 and 87-2852 (trade names; National Starch and Chemical Corporation), Nissetsu KP-77 and AS-370 (trade names; Nippon Carbide Industries Co., Ltd.). Its compounding amount is 5 to 99% by mass or, preferably, 10 to 90% by mass of the percutaneously absorbable patch and, when the compounding amount is made as such, there are big improvements in tackiness, adhesion to the skin for long time, percutaneous absorbability of the drug, pain upon peeling off, skin rash. Incidentally, when the compounding amount is less than 5% by mass, tackiness and adhesion to the skin for long time lower and pain upon peeling off, skin rash increase whereby the outcome is not favorable.

In the meanwhile, the percutaneousy absorbable patch of the present invention may, if necessary, be compounded with one or more member (s) of tackiness-giving agent, plasticizer.

With regard to the tackiness-giving agent, rosin ester, hydrogenated rosin ester, maleated rosin, alicyclic saturated hydrocarbon resin, terpene phenol may be used and its specific examples are Ester Gum A, AA-G, H or HP (trade names; Arakawa Chemical Industries, Ltd.), Hariester-L, S or P (trade names; Arakawa Chemical Industries, Ltd.), PINECRYSTAL KE-100 (trade name; Arakawa Chemical Industries, Ltd.), KE-311 (trade name; Arakawa Chemical Industries, Ltd.), Hercolyn D (trade name; Riken Hercules K. K.), Foral 85 or 105 (trade name; Riken Hercules K. K.), Stebelite ester 7 or 10 (trade name; Riken Hercules K. K.), Pentalyn 4820 or 4740 (trade name; Riken Hercules K. K.), Arkon P-85 or P-100 (trade name; Arakawa Chemical Industries, Ltd.). Its compounding amount is 5 to 50% by mass or, preferably, 10 to 40% by mass of the whole patch and, when the compounding amount is made as such, there are big improvements in tackiness, adhesion to the skin for long time, percutaneous absorbability of the drug, pain upon peeling off, skin rash. Incidentally, when the compounding amount is less than 5% by mass, tackiness and adhesion to the skin for long time lower whereby the outcome is not favorable. When it is more than 50% by mass, percutaneous absorbability of the drug, shape-holding property, lower and pain upon peeling off, skin rash, stickiness increase whereby the outcome is not favorable.

Examples of the plasticizer are petroleum-type oil (such as liquid paraffin, paraffin-type process oil, naphthene-type process oil and aromatic process oil), squalane, squalene, vegetable oil (such as olive oil, camellia oil, castor oil, tolu oil and peanut oil), dibasic acid ester (such as dibutyl phthalate and dioctyl phthalate), liquid rubber (such as liquid polybutene and liquid isoprene rubber) and isopropyl myristate. Among them, liquid paraffin, liquid polybutene and isopropyl myristate are particularly preferred. Its compounding amount is 10 to 70% by mass or, preferably, 15 to 60% by mass of the whole patch and, when the compounding amount is made as such, there are big improvements in tackiness, adhesion to the skin for long time, percutaneous absorbability of the drug, pain upon peeling off, skin rash. Incidentally, when the compounding amount is less than 10% by mass, pain upon peeling off becomes strong and the skin rash are resulted whereby the outcome is not favorable. When it is more than 70% by mass, cohesive force and shape-holding property lower and stickiness increase whereby the outcome is not favorable.

The percutaneously absorbable patch of the present invention may, if necessary, be further compounded with antioxidant (such as ascorbic acid, propyl gallate, butyl hydroxyanisole, dibutyl hydroxytoluene (BHT), nor-hydroxyguaiarrhetinic acid, tocopherol and tocopherol acetate), ultraviolet absorber (such as p-aminobenzoic acid, p-aminobenzoate, amyl p-dimemthylaminobenzoate, salicylate, methylanthranilate, umbelliferone, aesculin,benzylcinnamate, cinoxate, guaiazulene, urocanic acid, 2-(2-hydroxy-5-methylphenyl)benzotriazole, 4-methoxybenzophenone, 2-hydroxy-4-methoxybenzophenone, octabenzone, dioxybenzone, dihydroxydimethoxybenzophenone, sulisobenzone, benzoresorcinol, octyldimethyl p-aminobenzoate and ethylhexyl p-methoxycinamate), antibacterial (such as p-hydroxybenzoate, benzoic acid, benzoate, sorbic acid, sorbate, dehydroacetate, 4-isopropyl-3-methylphenol, 2-isopropyl-5-methylphenol, hinokitiol, cresol, 2,4,4-trichloro-2'-hydroxydiphenyl ether, 3,4,4'-trichlorocarbanide and chlorobutanol), filler (such as aluminum hydroxide, aluminum silicate hydrate, kaolin, titanium oxide, talc, zinc oxide, silica hydrate, magnesium carbonate, calcium hydrogen phosphate, magnesium silicate, diatomaceous earth, silicic acid anhydride, bentonite, sodium stearate, calcium stearate, potassium stearate, magnesium stearate, zinc stearate, magnesium myristate, zinc laurate and zinc undecylenoate), antihistaminic agent (such as isopentyl chloride, diphenhydramine hydrochloride, iproheptine hydrochloride, diphenylpyraline hydrochloride, cyproheptadine hydrochloride, triprolidine hydrochloride, promethazine hydrochloride, homochlorcyclizine hydrochloride, alimemazine hydrochloride, diphenhydramine tannate, diphenylpyraline theoclate, clemastine fumarate, chlorpheniramine maleate, dimethindene maleate and mequitazine), refreshing agent, perfume.

With regard to a support used for the percutaneously absorbable patch of the present invention, that which does not affect discharge and stability of the drug is preferred and stretching and non-stretching one are used. It is, for example, appropriately selected from film or sheet of polyethylene, polypropylene, polybutadiene, ethylene-vinyl acetate copolymer, poly(vinyl chloride), polyester, Nylon, polyurethane as well as layered product, porous product and foaming product thereof, paper, cloth, nonwoven fabric.

With regard to a release liner used for the percutaneously absorbable patch of the present invention, there is no particular limitation so far as it does not affect discharge and stability of the drug and it may be appropriately selected from release-coated paper, cellophane, polyethylene, polypropylene, polyester.

Examples

The present invention will now be more specifically illustrated by way of the following Examples. Incidentally, in Examples and Comparative Examples, the term "part (s)" means that/those by mass.

Example 1

| | |
|---|---|
| Styrene-isoprene-styrene block copolymer | 30.0 parts |
| (Trade name: Cariflex TR-1107) | |
| Liquid paraffin | 39.0 parts |
| Alicyclic saturated hydrocarbon resin | 20.0 parts |
| (Trade name: Arkon P-100) | |
| Diclofenac sodium | 4.0 parts |
| Diethylamine hydrochloride | 2.0 parts |
| Oleyl alcohol | 5.0 parts |

All of those components were added to toluene and, after the tacky substrate components were dissolved or dispersed, the mixture were applied onto a release liner so as to make the thickness after drying 100 μm. After drying, it was adhered to a support made of polyester to give a percutaneous absorbable patch of the present invention.

Example 2

| | |
|---|---|
| Styrene-isoprene-styrene block copolymer | 30.0 parts |
| (Trade name: Cariflex TR-1107) | |
| Liquid paraffin | 43.0 parts |
| Alicyclic saturated hydrocarbon resin | 20.0 parts |
| (Trade name: Arkon P-85) | |
| Diclofenac sodium | 1.0 parts |
| Diethylamine hydrochloride | 1.0 parts |
| Oleic acid | 5.0 parts |

All of those components were added to toluene and, after the tacky substrate components were dissolved or dispersed, the mixture were applied onto a release liner so as to make the thickness after drying 100 μm. After drying, it was adhered to a support made of polyester to give a percutaneous absorbable patch of the present invention.

Example 3

| Styrene-isoprene-styrene block copolymer | 20.0 parts |
| (Trade name: Cariflex TR-1107) | |
| Liquid paraffin | 31.0 parts |
| Alicyclic saturated hydrocarbon resin | 40.0 parts |
| (Trade name: Arkon P-100) | |
| Loxoprofen sodium | 3.0 parts |
| Ammonium chloride | 1.0 parts |
| Oleic acid | 5.0 parts |

All of those components were added to toluene and, after the tacky substrate components were dissolved or dispersed, the mixture were applied onto a release liner so as to make the thickness after drying 100 μm. After drying, it was adhered to a support made of polyester to give a percutaneous absorbable patch of the present invention.

Example 4

| Styrene-isoprene-styrene block copolymer | 25.0 parts |
| (Trade name: Cariflex TR-1107) | |
| Liquid paraffin | 52.0 parts |
| Alicyclic saturated hydrocarbon resin | 15.0 parts |
| (Trade name: Arkon P-85) | |
| Ketorolac tromethamine | 3.0 parts |
| n-Dodecyltetramethylammonium chloride | 2.0 parts |
| Oleyl alcohol | 3.0 parts |

All of those components were added to toluene and, after the tacky substrate components were dissolved or dispersed, the mixture were applied onto a release liner so as to make the thickness after drying 100 μm. After drying, it was adhered to a support made of polyester to give a percutaneous absorbable patch of the present invention.

Example 5

| Adhesive of an acrylate type | 66.0 parts |
| (Trade name: DURO-TAK 87-2194) | |
| Loxoprofen sodium | 20.0 parts |
| Diethylamine hydrochloride | 4.0 parts |
| Oleyl alcohol | 10.0 parts |

In a solution of an adhesive of an acrylate type were dissolved or dispersed all other components followed by applying onto a release liner so as to make the thickness after drying 50 μm. After drying, it was adhered to a support made of polyester to give a percutaneous absorbable patch of the present invention.

Example 6

| Adhesive of an acrylate type | 38.0 parts |
| (Trade name: DURO-TAK 87-2194) | |
| Isopropyl myristate | 30.0 parts |
| Diclofenac sodium | 20.0 parts |
| Ammonium chloride | 2.0 parts |

(continued)

| | |
|---|---|
| Oleic acid | 10.0 parts |

In a solution of an adhesive of an acrylate type were dissolved or dispersed all other components followed by applying onto a release liner so as to make the thickness after drying 50 μm. Afterdrying, it was adhered to a support made of polyester to give a percutaneous absorbable patch of the present invention.

Example 7

| | |
|---|---|
| Styrene-isoprene-styrene block copolymer (Trade name: Quintac 3530) | 15.0 parts |
| Liquid paraffin | 43.0 parts |
| Resin of a rosin type (Trade name: Stebelite ester 10) | 10.0 parts |
| Polyisobutylene (Trade name: Oppanol B-100) | 20.0 parts |
| Ketorolac tromethamine | 5.0 parts |
| Ammonium chloride | 3.0 parts |
| Polyoxyethylene (2) oleyl ether | 4.0 parts |

All of those components were added to toluene and, after the tacky substrate components were dissolved or dispersed, the mixture were applied onto a release liner so as to make the thickness after drying 150 μm. After drying, it was adhered to a support made of polyester to give a percutaneous absorbable patch of the present invention.

Example 8

| | |
|---|---|
| Styrene-isoprene-styrene block copolymer (Trade name: Quintac 3421) | 20.0 parts |
| Liquid paraffin | 37.0 parts |
| Resin of a rosin type (Trade name: Stebelite ester 7) | 20.0 parts |
| Polyisobutylene (Trade name: Oppanol B-200) | 15.0 parts |
| Diclofenac sodium | 5.0 parts |
| Diethylamine hydrochloride | 2.0 parts |
| Oleic acid | 1.0 part |

All of those components were added to toluene and, after the tacky substrate components were dissolved or dispersed, the mixture were applied onto a release liner so as to make the thickness after drying 100 μm. After drying, it was adhered to a support made of polyester to give a percutaneous absorbable patch of the present invention.

Example 9

| | |
|---|---|
| Styrene-isoprene-styrene block copolymer (Trade name: Cariflex TR-1107) | 25.0 parts |
| Liquid paraffin | 39.0 parts |
| Resin of a rosin type | 15.0 parts |

(continued)

| | |
|---|---|
| (Trade name: Foral 85) | |
| Polyisobutylene | 10.0 parts |
| (Trade name: Oppanol B-100) | |
| Diclofenac sodium | 5.0 parts |
| Diethylamine hydrochloride | 2.0 parts |
| Oleyl alcohol | 4.0 parts |

All of those components were added to toluene and, after the tacky substrate components were dissolved or dispersed, the mixture were applied onto a release liner so as to make the thickness after drying 100 μm. After drying, it was adhered to a support made of polyester to give a percutaneous absorbable patch of the present invention.

Example 10

| | |
|---|---|
| Styrene-isoprene-styrene block copolymer | 25.0 parts |
| (Trade name: Cariflex TR-1107) | |
| Liquid paraffin | 42.8 parts |
| Resin of a rosin type | 15.0 parts |
| (Trade name: Foral 85) | |
| Polyisobutylene | 10.0 parts |
| (Trade name: Oppanol B-100) | |
| Diclofenac sodium | 5.0 parts |
| Diethylamine hydrochloride | 2.0 parts |
| Oleyl alcohol | 0.2 part |

All of those components were added to toluene and, after the tacky substrate components were dissolved or dispersed, the mixture were applied onto a release liner so as to make the thickness after drying 100 μm. After drying, it was adhered to a support made of polyester to give a percutaneous absorbable patch of the present invention.

Example 11

| | |
|---|---|
| Styrene-isoprene-styrene block copolymer | 25.0 parts |
| (Trade name: Cariflex TR-1107) | |
| Liquid paraffin | 18.0 parts |
| Resin of a rosin type | 15.0 parts |
| (Trade name: Foral 85) | |
| Polyisobutylene | 10.0 parts |
| (Trade name: Oppanol B-100) | |
| Diclofenac sodium | 5.0 parts |
| Diethylamine hydrochloride | 2.0 parts |
| Oleyl alcohol | 25.0 parts |

All of those components were added to toluene and, after the tacky substrate components were dissolved or dispersed, the mixture were applied onto a release liner so as to make the thickness after drying 100 μm. After drying, it was adhered to a support made of polyester to give a percutaneous absorbable patch of the present invention.

Example 12

| Styrene-isoprene-styrene block copolymer (Trade name: Cariflex TR-1111) | 20.0 parts |
|---|---|
| Liquid paraffin | 31.0 parts |
| Resin of a rosin type (Trade name: KE-100) | 20.0 parts |
| Polyisobutylene (Trade name: Vistanex MML-80) | 15.0 parts |
| Diclofenac sodium . | 5.0 parts |
| Diethylamine hydrochloride | 2.0 parts |
| Oleic acid | 7.0 parts |

All of those components were added to toluene and, after the tacky substrate components were dissolved or dispersed, the mixture were applied onto a release liner so as to make the thickness after drying 100 μm. After drying, it was adhered to a support made of polyester to give a percutaneous absorbable patch of the present invention.

Example 13

| Styrene-isoprene-styrene block copolymer (Trade name: Quintac 3570 C) | 35.0 parts |
|---|---|
| Adhesive of an acrylate type (Trade name: DURO-TAK 87-2516) | 47.0 parts |
| Diclofenac sodium | 10.0 parts |
| Ammonium chloride | 3.0 parts |
| Oleyl alcohol | 5.0 parts |

In a solution of an adhesive of an acrylate type were dissolvedor dispersed all other components followed by applying onto a release liner so as to make the thickness after drying 100 μm. After drying, it was adhered to a support made of polyester to give a percutaneous absorbable patch of the present invention.

Example 14

| Styrene-isoprene-styrene block copolymer (Trade name: JSR SIS-5000) | 35.0 parts |
|---|---|
| Adhesive of an acrylate type (Trade name: DURO-TAK 87-2516) | 47.0 parts |
| Diclofenac sodium | 10.0 parts |
| Ammonium chloride | 3.0 parts |
| Oleic acid | 5.0 parts |

In a solution of an adhesive of an acrylate type were dissolvedor dispersed all other components followed by applying onto a release liner so as to make the thickness after drying 50 μm. Afterdrying, it was adhered to a support made of polyester to give a percutaneous absorbable patch of the present invention.

Example 15

| Polyisobutylene (Trade name: Vistanex LM-MS) | 20.0 parts |
|---|---|

(continued)

| | |
|---|---|
| Adhesive of an acrylate type | 65.5 parts |
| (Trade name: DURO-TAK 87-2516) | |
| Diclofenac sodium | 10.0 parts |
| Diethylamine hydrochloride | 4.0 parts |
| Oleic acid | 0.5 part |

In a solution of an adhesive of an acrylate type were dissolved or dispersed diclofenac sodium, oleic acid and diethylamine hydrochloride, a solution of polyisobutylene dissolved in toluene was added thereto and the mixture was homogeneously stirred and applied onto a release liner so as to make the thickness after drying 100 $\mu$m. After drying, it was adhered to a support made of polyester to give a percutaneous absorbable patch of the present invention.

Example 16

| | |
|---|---|
| Polyisobutylene | 20.0 parts |
| (Trade name: Tetrax 4T) | |
| Adhesive of an acrylate type | 65.5 parts |
| (Trade name: DURO-TAK 87-2516) | |
| Diclofenac sodium | 10.0 parts |
| Diethylamine hydrochloride | 4.0 parts |
| Oleyl alcohol | 0.5 part |

In a solution of an adhesive of an acrylate type were dissolved or dispersed diclofenac sodium, oleyl alcohol and diethylamine hydrochloride, a solution of polyisobutylene dissolved in toluene was added thereto and the mixture was homogeneously stirred and applied onto a release liner so as to make the thickness after drying 50 $\mu$m. After drying, it was adhered to a support made of polyester to give a percutaneous absorbable patch of the present invention.

Example 17

| | |
|---|---|
| Styrene-isoprene-styrene block copolymer | 25.0 parts |
| (Trade name: Cariflex TR-1107) | |
| Liquid paraffin | 40.0 parts |
| Resin of a rosin type | 15.0 parts |
| (Trade name: Foral 85) | |
| Polyisobutylene | 10.0 parts |
| (Trade name: Oppanol B-100) | |
| Diclofenac sodium | 5.0 parts |
| Diethylamine hydrochloride | 2.0 parts |
| Dipropylene glycol | 3.0 parts |

All of those components were added to toluene and, after the tacky substrate components were dissolved or dispersed, the mixture were applied onto a release liner so as to make the thickness after drying 100 $\mu$m. After drying, it was adhered to a support made of polyester to give a percutaneous absorbable patch of the present invention.

Example 18

| | |
|---|---|
| Styrene-isoprene-styrene block copolymer | 25.0 parts |
| (Trade name: Cariflex TR-1107) | |

(continued)

| Liquid paraffin | 42.8 parts |
| Resin of a rosin type | 15.0 parts |
| (Trade name: Foral 85) | |
| Polyisobutylene | 10.0 parts |
| (Trade name: Oppanol B-100) | |
| Diclofenac sodium | 5.0 parts |
| Diethylamine hydrochloride | 2.0 parts |
| Dipropylene glycol | 0.2 part |

All of those components were added to toluene and, after the tacky substrate components were dissolved or dispersed, the mixture were applied onto a release liner so as to make the thickness after drying 100 $\mu$m. After drying, it was adhered to a support made of polyester to give a percutaneous absorbable patch of the present invention.

Example 19

| Styrene-isoprene-styrene block copolymer | 25.0 parts |
| (Trade name: Cariflex TR-1107) | |
| Liquid paraffin | 13.0 parts |
| Resin of a rosin type | 15.0 parts |
| (Trade name: Foral 85) | |
| Polyisobutylene | 10.0 parts |
| (Trade name: Oppanol B-100) | |
| Diclofenac sodium | 5.0 parts |
| Diethylamine hydrochloride | 2.0 parts |
| Dipropylene glycol | 30.0 parts |

All of those components were added to toluene and, after the tacky substrate components were dissolved or dispersed, the mixture were applied onto a release liner so as to make the thickness after drying 100 $\mu$m. After drying, it was adhered to a support made of polyester to give a percutaneous absorbable patch of the present invention.

Example 20

| Styrene-isoprene-styrene block copolymer | 30.0 parts |
| (Trade name: Cariflex TR-1107) | |
| Liquid paraffin | 34.0 parts |
| Alicyclic.saturated hydrocarbon resin | 10.0 parts |
| (Trade name: Arkon P-85) | |
| Polyisobutylene | 15.0 parts |
| (Trade name: Oppanol B-100) | |
| Diclofenac sodium | 3.0 parts |
| Ammonium chloride | 1.0 parts |
| Triethylene glycol | 7.0 parts |

All of those components were added to toluene and, after the tacky substrate components were dissolved or dispersed, the mixture were applied onto a release liner so as to make the thickness after drying 100 $\mu$m. After drying, it was adhered to a support made of polyester to give a percutaneous absorbable patch of the present invention.

Example 21

| | |
|---|---|
| Styrene-isoprene-styrene block copolymer (Trade name: Cariflex TR-1107) | 30.0 parts |
| Liquid paraffin | 36.0 parts |
| Resin of a rosin type (Trade name: KE-311) | .10.0 parts |
| Polyisobutylene (Trade name: Oppanol B-100) | 15.0 parts |
| Diclofenac sodium | 3.0 parts |
| Diethylamine hydrochloride | 1.0 part |
| Polyethylene glycol 400 | 5.0 parts |

All of those components were added to toluene and, after the tacky substrate components were dissolved or dispersed, the mixture were applied onto a release liner so as to make the thickness after drying 100 μm. After drying, it was adhered to a support made of polyester to give a percutaneous absorbable patch of the present invention.

Example 22

| | |
|---|---|
| Styrene-isoprene-styrene block copolymer (Trade name: Cariflex TR-1107) | 30.0 parts |
| Liquid paraffin | 10.0 parts |
| Adhesive of an acrylate type (Trade name: DURO-TAK 87-2516) | 46.0 parts |
| Diclofenac sodium | 3.0 parts |
| Ammonium chloride | 1.0 part |
| Polyoxyethylene lauryl ether | 10.0 parts |

In a solution of an adhesive of an acrylate type were dissolved or dispersed all other components followed by applying onto a release liner so as to make the thickness after drying 50μm. After drying, it was adhered to a supportmade of polyester to give a percutaneous absorbable patch of the present invention.

Example 23

| | |
|---|---|
| Polyisobutylene (Trade name: Tetrax 4T) | 15.0 parts |
| Isopropyl myristate | 20.0 parts |
| Adhesive,of an acrylate type (Trade name: DURO-TAK 87-2516) | 46.0 parts |
| Diclofenac sodium | 3.0 parts |
| Diethylamine hydrochloride | 1.0 part |
| Polyoxyethylene sorbitan monooleate | 15.0 parts |

In a solution of an adhesive of an acrylate type were dissolved or dispersed diclofenac sodium, polyoxyethylene sorbitan monooleate and diethylamine hydrochloride, a solution of polyisobutylene dissolved in toluene was added thereto and the mixture was homogeneously stirred and applied onto a release liner so as to make the thickness after drying 50 μm. After drying, it was adhered to a support made of polyester to give a percutaneous absorbable patch of the present invention.

Example 24

| | |
|---|---|
| Styrene-isoprene-styrene block copolymer (Trade name: Cariflex TR-1107) | 30.0 parts |
| Liquid paraffin | 30.0 parts |
| Alicyclic saturated hydrocarbon resin (Trade name: Arkon P-100) | 20.0 parts |
| Polyisobutylene (Trade name: Oppanol B-100) | 15.0 parts |
| Diclofenac sodium | 3.0 parts |
| Ammonium chloride | 1.0 part |
| Crotamiton | 1.0 part |

All of those components were added to toluene and, after the tacky substrate components were dissolved or dispersed, the mixture were applied onto a release liner so as to make the thickness after drying 100 μm. After drying, it was adhered to a support made of polyester to give a percutaneous absorbable patch of the present invention.

Example 25

| | |
|---|---|
| Styrene-isoprene-styrene block copolymer (Trade name: Cariflex TR-1107) | 25.0 parts |
| Liquid paraffin | 35.0 parts |
| Resin of a rosin type (Trade name: Foral 85) | 15.0 parts |
| Polyisobutylene (Trade name: Oppanol B-100) | 10.0 parts |
| Diclofenac sodium | 5.0 parts |
| Diethylamine hydrochloride | 2.0 parts |
| Oleyl alcohol | 5.0 parts |
| Dipropylene glycol | 3.0 parts |

All of those components were added to toluene and, after the tacky substrate components were dissolved or dispersed, the mixture were applied onto a release liner so as to make the thickness after drying 100 μm. After drying, it was adhered to a support made of polyester to give a percutaneous absorbable patch of the present invention.

Example 26

| | |
|---|---|
| Styrene-isoprene-styrene block copolymer (Trade name: Cariflex TR-1107) | 25.0 parts |
| Liquid paraffin | 29.0 parts |
| Alicyclic saturated hydrocarbon resin (Trade name: Arkon P-85) | 15.0 parts |
| Polyisobutylene (Trade name: Oppanol B-100) | 15.0 parts |
| Diclofenac sodium | 3.0 parts |
| Diethylamine hydrochloride | 1.0 part |
| Oleic acid | 5.0 parts |
| Triethylene glycol | 7.0 parts |

All of those components were added to toluene and, after the tacky substrate components were dissolved or dispersed, the mixture were applied onto a release liner so as to make the thickness after drying 100 μm. After drying, it was adhered to a support made of polyester to give a percutaneous absorbable patch of the present invention.

Example 27

| | |
|---|---|
| Styrene-isoprene-styrene block copolymer | 20.0 parts |
| (Trade name: Cariflex TR-1107) | |
| Liquid paraffin | 31.0 parts |
| Resin of a rosin type | 20.0 parts |
| (Trade name: Foral 105) | |
| Polyisobutylene | 15.0 parts |
| (Trade name: Oppanol B-100) | |
| Diclofenac sodium | 3.0 parts |
| Ammonium chloride | 1.0 part |
| Oleic acid | 5.0 parts |
| Polyethylene glycol 400 | 5.0 parts |

All of those components were added to toluene and, after the tacky substrate components were dissolved or dispersed, the mixture were applied onto a release liner so as to make the thickness after drying 100 μm. After drying, it was adhered to a support made of polyester to give a percutaneous absorbable patch of the present invention.

Example 28

| | |
|---|---|
| Styrene-isoprene-styrene block copolymer | 10.0 parts |
| (Trade name: Cariflex TR-1107) | |
| Liquid paraffin | 21.0 parts |
| Adhesive of an acrylate type | 50.0 parts |
| (Trade name: DURO-TAK 87-2516) | |
| Diclofenac sodium | 3.0 parts |
| Ammonium chloride | 1.0 part |
| Oleyl alcohol | 5.0 parts |
| Polyoxyethylene lauryl ether | 10.0 parts |

In a solution of an adhesive of an acrylate type were dissolved or dispersed all other components followed by applying onto a release liner so as to make the thickness after drying 50μm. After drying, it was adhered to a supportmade of polyester to give a percutaneous absorbable patch of the present invention.

Example 29

| | |
|---|---|
| Polyisobutylene | 16.0 parts |
| (Trade name: Tetrax 4T) | |
| Isopropyl myristate | 30.0 parts |
| Adhesive of an acrylate type | 30.0 parts |
| (Trade name: DURO-TAK 87-2516) | |
| Diclofenac sodium | 3.0 parts |
| Diethylamine hydrochloride | 1.0 part |
| Oleyl alcohol | 5.0 parts |

(continued)

| | |
|---|---|
| Polyoxyethylene sorbitan monooleate | 15.0 parts |

In a solution of an adhesive of an acrylate type were dissolved or dispersed diclofenac sodium, polyoxyethylene sorbitan monooleate and diethylamine hydrochloride, a solution of polyisobutylene dissolved in toluene was added thereto and the mixture was homogeneously stirred and applied onto a release liner so as to make the thickness after drying 50 $\mu$m. After drying, it was adhered to a support made of polyester to give a percutaneous absorbable patch of the present invention.

Example 30

| | |
|---|---|
| Styrene-isoprene-styrene block copolymer (Trade name: Cariflex TR-1107) | 30.0 parts |
| Liquid paraffin | 30.0 parts |
| Resin of a rosin type (Trade name: Foral 85) | 10.0 parts |
| Alicyclic saturated hydrocarbon resin (Trade name: Arkon P-85). | 10.0 parts |
| Polyisobutylene (Trade name: Oppanol B-100) | 10.0 parts |
| Diclofenac sodium | 3.0 parts |
| Ammonium chloride | 1.0 part |
| Oleyl alcohol | 5.0 parts |
| Crotamiton | 1.0 part |

All of those components were added to toluene and, after the tacky substrate components were dissolved or dispersed, the mixture were applied onto a release liner so as to make the thickness after drying 100 $\mu$m. After drying, it was adhered to a support made of polyester to give a percutaneous absorbable patch of the present invention.

Comparative Example 1

| | |
|---|---|
| Styrene-isoprene-styrene block copolymer (Trade name: Cariflex TR-1107) | 25.0 parts |
| Liquid paraffin | 45.0 parts |
| Resin of a rosin type (Trade name: Foral 85) | 15.0 parts |
| Polyisobutylene (Trade name: Oppanol B-100) | 10.0 parts |
| Diclofenac sodium | 5.0 parts |

All of those components were added to toluene and, after the tacky substrate components were dissolved or dispersed, the mixture were applied onto a release liner so as to make the thickness after drying 100 $\mu$m. After drying, it was adhered to a support made of polyester to give a percutaneous absorbable patch of the present invention.

Comparative Example 2

| | |
|---|---|
| Styrene-isoprene-styrene block copolymer (Trade name: Cariflex TR-1107) | 25.0 parts |

(continued)

| Liquid paraffin | 43.0 parts |
|---|---|
| Resin of a rosin type | 15.0 parts |
| (Trade name: Foral 85) | |
| Polyisobutylene | 10.0 parts |
| (Trade name: Oppanol B-100) | |
| Diclofenac sodium | 5.0 parts |
| Diethylamine hydrochloride | 2.0 parts |

All of those components were added to toluene and, after the tacky substrate components were dissolved or dispersed, the mixture were applied onto a release liner so as to make the thickness after drying 100 $\mu$m. After drying, it was adhered to a support made of polyester to give a percutaneous absorbable patch of the present invention.

Comparative Example 3 (Salt of ammonium compound being uncompounded)

| Styrene-isoprene-styrene block copolymer | 25.0 parts |
|---|---|
| (Trade name: Cariflex TR-1107) | |
| Liquid paraffin | 41.0 parts |
| Resin of a rosin type | 15.0 parts |
| (Trade name: Foral 85) | |
| Polyisobutylene | 10.0 parts |
| (Trade name: Oppanol B-100) | |
| Diclofenac sodium | 5.0 parts |
| Oleyl alcohol | 4.0 parts |

All of those components were added to toluene and, after the tacky substrate components were dissolved or dispersed, the mixture were applied onto a release liner so as to make the thickness after drying 100 $\mu$m. After drying, it was adhered to a support made of polyester to give a percutaneous absorbable patch of the present invention.

Comparative Example 4

| Styrene-isoprene-styrene block copolymer | 20.0 parts |
|---|---|
| (Trade name: Cariflex TR-1107) | |
| Liquid paraffin | 36.0 parts |
| Alicyclic saturated hydrocarbon resin | 40.0 parts |
| (Trade name: Arkon P-85) | |
| Loxoprofen sodium | 3.0 parts |
| Ammonium chloride | 1.0 part |

All of those components were added to toluene and, after the tacky substrate components were dissolved or dispersed, the mixture were applied onto a release liner so as to make the thickness after drying 100 $\mu$m. After drying, it was adhered to a support made of polyester to give a percutaneous absorbable patch of the present invention.

Comparative Example 5

| Styrene-isoprene-styrene block copolymer | 25.0 parts |
|---|---|
| (Trade name: Cariflex TR-1107) | |

(continued)

| | |
|---|---|
| Liquid paraffin | 55.0 parts |
| Alicyclic saturated hydrocarbon resin (Trade name: Arkon P-85) | 15.0 parts |
| Ketorolac tromethamine | 3.0 parts |
| n-Dodecyltetramethylammonium chloride | 2.0 parts |

All of those components were added to toluene and, after the tacky substrate components were dissolved or dispersed, the mixture were applied onto a release liner so as to make the thickness after drying 100 $\mu$m. After drying, it was adhered to a support made of polyester to give a percutaneous absorbable patch of the present invention.

Test Example 1 (*In vitro* skin permeation test)

Skin of the back of hairless mouse (8 weeks age; female) was excised, fat at the side of dermis was carefully removed and the skin was attached to a flow-through cell in which water of 37°C was circulated in the outer circumference of a receptor layer where the dermis side was the receptor layer. A percutaneously absorbable patch of an Example or a Comparative Example was applied to the horny layer thereof and a phosphate buffer of pH 7.4 was applied to the receptor layer at the rate of 1 ml/hour and sampling was conducted every four hours until 24th hour. Flow rate of the solution obtained from each sampling was precisely measured, drug concentration was measured by means of a high performance liquid chromatography, permeated rate every four hours was calculated and the skin permeation rate at the stationary state was determined according to the following formula.

$$\text{Skin Permeation Rate } (\mu g/cm^2/hr) = [\text{Sample concentration}$$

$$(\mu g/ml) \times \text{Flow rate } (ml)]/\text{Area to which the Preparation was}$$

$$\text{Applied } (cm^2) \times 4 \text{ hr}$$

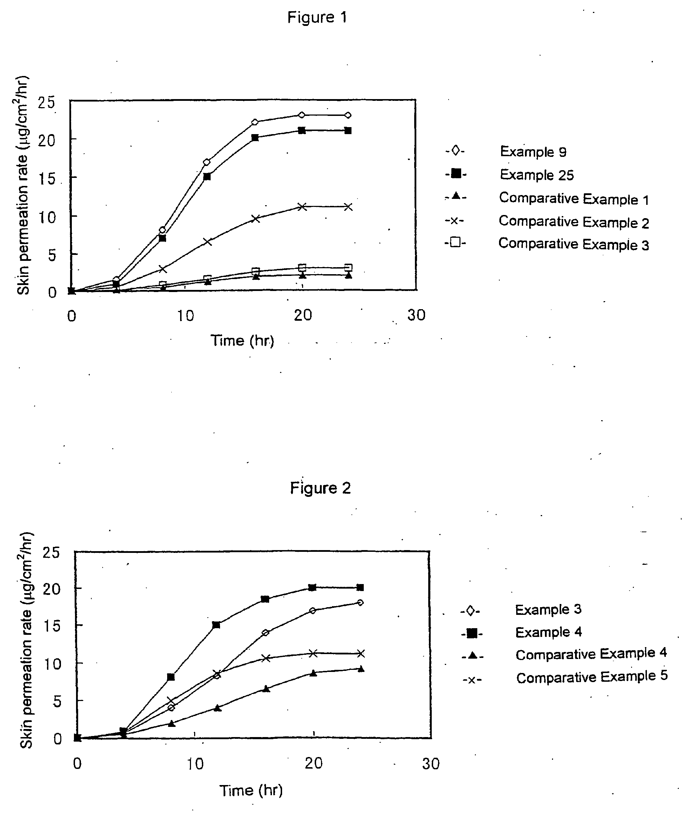

The result is shown in Fig. 1 and Fig. 2.
In Fig. 1, -◇-, -■-, -▲-, -✕- and -□- show the results for percutaneously absorbable patches of Example 9, Example 25, Comparative Example 1, Comparative Example 2 and Comparative Example 3, respectively.
In Fig. 2, -◇-, -■-, -▲- and -✕- show the results for percutaneously absorbable patches of Example 3, Example 4, Comparative Example 4 and Comparative Example 5, respectively.
As being apparent from Fig. 1 and Fig. 2, the percutaneously absorbable patches of Examples 3, 4, 9 and 25 which are the percutaneously absorbable patches of the present invention show higher percutaneous absorbability than those of Comparative Examples 1 to 5.

Test Example 2 (Cohesive force test)

A sample which was previously allowed to stand for more than 30 minutes in a constant-temperature chamber of 25°C was cut out in a size of a sample-holding ring followed removing a liner therefrom, adhered to the sample-holding ring making the adhesive surface downside and placed on a sample stand. After that, a probe shaft was ascended at a constant rate and the adhesive side of the sample was contacted to a probe of 5 mm diameter made of bakelite with a pressure load of 100 g/cm$^2$ for one second. Then the probe shaft was descended at the rate of 0. 5 cm/sec and the probe was detached from the adhesive side. The force required therefor was measured as a cohesive force. Cohesive force of each sample was measured immediately after manufacture and after preservation for one month at 40°C and the cohesive force of the initial stage and stability of the cohesive with a lapse of time were evaluated. The result is shown in Table 1.

Table 1

| | Cohesive Force Test | |
|---|---|---|
| | Cohesive Force (g) | |
| | Immediately after Manufacture | After Preserved at 40°C for 1 Month |
| Example 3 | 98 | 94 |
| Example 8 | 90 | 91 |
| Example 9 | 95 | 92 |
| Example 11 | 42 | 18 |
| Example 19 | 35 | 12 |
| Example 21 | 85 | 85 |
| Example 25 | 83 | 86 |

Test Example 3 (Observation of Crystallization)

After the sample was preserved for one month in a constant-temperature and constant-humidity chamber of 40°C and 75%, it was observed whether crystallization took place in the substrate. The result is shown in Table 2.

Table 2

| | Crystallization Observation |
|---|---|
| Example 17 | not crystallized |
| Example 20 | not crystallized |
| Example 21 | not crystallized |
| Example 25 | not crystallized |
| Example 26 | not crystallized |
| Comparative Example 2 | crystallized |
| Comparative Example 5 | crystallized |

As being apparent from Table 2, in the percutaneously absorbable patches of Comparative Example 2 and Comparative Example 5 where no solubilizing agent was contained in the adhesive substrate layer, separation of crystals was noted in the substrate after preserved for one month while, in the percutaneously absorbable patches of Examples 17, 20, 21, 25 and 26 where a solubilizing agent was contained in the adhesive substrate layer, no separation of crystals was noted at all.

Industrial Applicability

As mentioned hereinabove, in accordance with the present invention, a salt of ammonium compound and an absorption enhancer and/or a solubilizing agent are compounded in an adhesive substrate layer containing an anti-inflammatory agent form of a salt whereupon it is now possible to prepare a percutaneously absorbable patch where a percutaneous absorbability of an anti-inflammatory agent in the form of a salt is significantly improved and no crystal of the drug is separated out even when preserved for a long period but stability is enhanced. Thus, there is achieved a remarkable advantage that a useful pharmaceutical preparation which has not been available yet is able to be provided.

**Claims**

1.  A percutaneously absorbable patch having an adhesive substrate layer which contains an anti-inflammatory agent in the form of a salt, a salt of an ammonium compound; and comprising an absorption enhancer chosen from an unsaturated fatty acid, oleyl alcohol and polyoxy ethylene oleyl ether and/or a solubilizing agent selected from the group consisting of a polyhydric alcohol, a nonionic surface-active agent and crotamiton.

2. A percutaneously absorbable patch according to claim 1, wherein the amount of salt of the ammonium compound is within the range of 0.5-fold mol to 7-fold mol to the anti-inflammatory agent in the form of a salt.

3. A percutaneously absorbable patch according to either claim 1 or claim 2, wherein the amount of the absorption enchancer is 0.5 to 15% by mass.

4. A percutaneously absorbable patch according to any one of claims 1 to 3, wherein the absorption enhancer is oleic acid and/or oleyl alcohol.

5. A percutaneously absorbable patch according to any one of claims 1 to 4, wherein the amount of the solubilizing agent is 0.5 to 20% by mass.

6. A percutaneously absorbable patch according to any one of claims 1 to 4, wherein the solubilizing agent is crotamiton.

7. A percutaneously absorbable patch according to any one of claims 1 to 6, wherein the adhesive substrate is one or more polymer(s) selected from the group consisting of styrene-isoprene-styrene block copolymer, polyisobutylene and an adhesive agent of an acrylate type.

8. A percutaneously absorbable patch according to any one of claims 1 to 7, wherein the anti-inflammatory agent is an acidic drug.

9. A percutaneously absorbable patch according to any one of claims 1 to 7, wherein the anti-inflammatory agent in the form of a salt is a salt of diclofenac.

10. A percutaneously absorbable patch according to any one of claims 1 to 7, wherein the anti-inflammatory agent in the form of a salt is diclofenac sodium or diclofenac potassium.

11. A percutaneously absorbable patch according to any one of claims 1 to 10, wherein the salt of ammonium compound is ammonium chloride and/or diethylamine hydrochloride.

**Patentansprüche**

1. Perkutan absorbierbares Pflaster mit einer klebenden Substratschicht, welche ein entzündungshemmendes Mittel in der Form eines Salzes, ein Salz einer Ammonium-Verbindung enthält; und umfassend einen Absorptionsverstärker, ausgewählt aus einer ungesättigten Fettsäure, Oleylalkohol und Polyoxyethylenoleylether, und/oder ein Solubilisierungsmittel, ausgewählt aus der Gruppe, bestehend aus einem polyhydrischen Alkohol, einem nichtionischen oberflächenaktiven Mittel und Crotamiton.

2. Perkutan absorbierbares Pflaster nach Anspruch 1, worin die Menge des Salzes der Ammonium-Verbindung innerhalb des Bereichs von 0,5-fachen Mol bis 7-fachen Mol relativ zu dem entzündungshemmenden Mittel in der Form eines Salzes liegt.

3. Perkutan absorbierbares Pflaster entweder nach Anspruch 1 oder Anspruch 2, worin die Menge des Absorptionsverstärkers 0,5 bis 15 Massenprozent beträgt.

4. Perkutan absorbierbares Pflaster nach einem der Ansprüche 1 bis 3, worin der Absorptionsverstärker Oleinsäure und/oder Oleylalkohol ist.

5. Perkutan absorbierbares Pflaster nach einem der Ansprüche 1 bis 4, worin die Menge des Solubilisierungsmittels 0,5 bis 20 Massenprozent beträgt.

6. Perkutan absorbierbares Pflaster nach einem der Ansprüche 1 bis 4, worin das Solubilisierungsmittel Crotamiton ist.

7. Perkutan absorbierbares Pflaster nach einem der Ansprüche 1 bis 6, worin das Klebesubstrat aus ein oder mehreren Polymeren besteht, ausgewählt aus der Gruppe, bestehend aus Styrol-Isopren-Styrol-Blockcopolymer, Polyisobutylen und einem Klebstoff eines Acrylat-Typs.

**8.** Perkutan absorbierbares Pflaster nach einem der Ansprüche 1 bis 7, worin das entzündungshemmende Mittel ein saurer Wirkstoff ist.

**9.** Perkutan absorbierbares Pflaster nach einem der Ansprüche 1 bis 7, worin das entzündungshemmende Mittel in der Form eines Salzes ein Salz von Diclofenac ist.

**10.** Perkutan absorbierbares Pflaster nach einem der Ansprüche 1 bis 7, worin das entzündungshemmende Mittel in der Form eines Salzes Diclofenac-Natrium oder Diclofenac-Kalium ist.

**11.** Perkutan absorbierbares Pflaster nach einem der Ansprüche 1 bis 7, worin das Salz der Ammonium-Verbindung Ammoniumchlorid und/oder Diethylaminhydrochlorid ist.

**Revendications**

**1.** Timbre à absorption percutanée ayant une couche substrat adhésive qui contient un agent anti-inflammatoire sous la forme d'un sel, d'un sel d'un composé d'ammonium ; et comprenant un activateur d'absorption choisi parmi un acide gras insaturé, l'alcool oléylique et un éther oléylique de polyoxyéthylène et/ou un agent solubilisant choisi dans le groupe constitué par un alcool polyhydrique, un agent tensioactif non ionique et le crotamiton.

**2.** Timbre à absorption percutanée selon la revendication 1, dans lequel la quantité de sel du composé d'ammonium est dans la gamme de 0,5 fois molaire à 7 fois molaire par rapport à l'agent anti-inflammatoire sous la forme d'un sel.

**3.** Timbre à absorption percutanée selon la revendication 1 ou la revendication 2, dans lequel la quantité de l'activateur d'absorption est de 0,5 à 15% en masse.

**4.** Timbre à absorption percutanée selon l'une quelconque des revendications 1 à 3, dans lequel l'activateur d'absorption est l'acide oléique et/ou l'alcool oléylique.

**5.** Timbre à absorption percutanée selon l'une quelconque des revendications 1 à 4, dans lequel la quantité de l'agent solubilisant est de 0,5 à 20 % en masse.

**6.** Timbre à absorption percutanée selon l'une quelconque des revendications 1 à 4, dans lequel l'agent solubilisant est le crotamiton.

**7.** Timbre à absorption percutanée selon l'une quelconque des revendications 1 à 6, dans lequel le substrat adhésif est un ou plusieurs polymère(s) choisi(s) dans le groupe constitué par un copolymère séquencé styrène-isoprène-styrène, un polyisobutylène et un agent adhésif d'un type acrylate.

**8.** Timbre à absorption percutanée selon l'une quelconque des revendications 1 à 7, dans lequel l'agent anti-inflammatoire est une substance médicamenteuse acide.

**9.** Timbre à absorption percutanée selon l'une quelconque des revendications 1 à 7, dans lequel l'agent anti-inflammatoire sous la forme d'un sel est un sel de diclofénac.

**10.** Timbre à absorption percutanée selon l'une quelconque des revendications 1 à 7, dans lequel l'agent anti-inflammatoire sous la forme d'un sel est le diclofénac sodium ou le diclofénac potassium.

**11.** Timbre à absorption percutanée selon l'une quelconque des revendications 1 à 10, dans lequel le sel de composé d'ammonium est le chlorure d'ammonium et/ou le chlorhydrate de diéthylamine.

Figure 1

| | |
|---|---|
| -◇- | Example 9 |
| -■- | Example 25 |
| -▲- | Comparative Example 1 |
| -x- | Comparative Example 2 |
| -□- | Comparative Example 3 |

Figure 2

| | |
|---|---|
| -◇- | Example 3 |
| -■- | Example 4 |
| -▲- | Comparative Example 4 |
| -x- | Comparative Example 5 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7047535 B
- JP 5155762 A
- JP 3067043 B
- JP 9048739 A
- JP 10512245 A
- WO 0105381 A